# EUROPEAN PATENT APPLICATION

(11) **EP 3 845 138 A1**
(43) Date of publication of application: **07.07.2021**
(21) Application number: 21020002.8
(22) Date of filing: 01.01.2021
(51) Int. Cl.: A61B 17/04, A61B 17/06, A61F 2/24, A61B 17/00

(54) **ANCHOR TO REPAIR A MITRAL VALVE PROLAPSE**

(30) Priority: 02.01.2020 DE 202020000044 U
(71) Applicant: Vusal, Hajiyev, 10707 Berlin (DE)
(72) Inventor: Vusal, Hajiyev, 10707 Berlin (DE)
(74) Representative: Toningerova, Daniela

(57) **Abstract**

Anchor (1) for repair of mitral valve prolapse by fixing mitral valve leaflets into the physiological position. The anchor (1) having an elongated shape is provided with at least one aperture (2) for passing of a thread (3). The anchor can have a shape of cylinder, ellipsoid or prism. The anchor (1) can be also made by a roll of an elastic sheet. The anchor (1) provided with the thread (3) is pushed into the left ventricle (8) through the hollow needle (5) using a guide wire (4). After removing the hollow needle (5) the anchor captures the mitral valve leaflet (7) and transfers it into the physiological position and fix it in this position by securing the thread (3) to the apex of the heart.

## Description

### Technical field

The invention relates to the general technical field of medical devices and in particular to an anchor to repair a mitral valve prolapse by fixing mitral valve leaflet in the physiological position and method of use thereof.

### Background

The heart valve disease is a common heart defect that needs to be treated surgically. The valve serves as a valve between the atrium and the ventricle to prevent blood from flowing back into the atrium. The tricuspid valve is located between the right ventricle and the atrium, the mitral (bicuspid) valve is located between the left ventricle and the atrium, and is responsible for regulating the flow of oxygenated blood from the lungs. Oxygenated blood flows from the left atrium through the mitral valve to the left ventricle, the blood pressure from the atrial side opens the mitral valve. The mitral valve is then closed by closing the valve leaflets, when blood is expelled from the left ventricle into the aorta.

A common disease of the mitral valve is mitral regurgidation (MR) or insufficiency, when the valve leaflets do not close properly. The most common cause of MR is prolapse of one or both mitral valve leaflets. In this case, the mitral valve can be surgically replaced or reconstructed.

Currently, it is preferred to perform a conservation operation instead of the mitral valve replacement. Patients are not in the risks of thrombosis or endocarditis of the implanted valve.

One document **(D1)** describes use of artificial chordae to correct position of mitral valve leaflets.

Another document **(D2)** describes set of 4 artificial chordae loops made from Gore - Tex for fixing the prolapsing leaflet of the mitral valve.

These procedures usually require an open heart surgery, opening the chest, stopping the heart and connecting the patient to the extracorporeal circulation. The open heart surgery can lead to significant complications such as stroke, heart failure or myocardial infarction, with a relatively high mortality, especially in elderly and seriously ill patients.

Therefore mini-invasive surgeries performed on a beating heart using microscopic video control have been increasingly used during the last decade.

Another document **(D3)** describes a surgical device Mitraclip, which is used for catheter procedures of the heart valve surgery.

EP3263049 **(D4)** discloses a device for repair a heart valve leaflet comprising a catheter assembly provided with a capture mechanism rotatably coupled to a plication mechanism and comprising a clip and a safety tether coupled to both the clip and the catheter assembly.

### Summary of the invention

The present invention relates to an anchor to repair a mitral valve prolapse. The anchor is adapted to pass into the heart through a hollow needle, in order to capture a mitral valve leaflet, transfer it into the physiological position and fix it in this position.

The anchor according to invention has an elongated shape, which is adapted for longitudinal passage through the hollow needle by means of a guide wire and it is provided with at least one aperture for passing of a thread. The elongated shape may be a cylinder, an ellipsoid or a prism. It may be a rectangular prism, a pentagonal prism or more angular prism, trigrammic prism or more-grammic prism. The thread is preferably a surgical thread. The width (diameter) of the anchor is smaller than the inner diameter of the hollow needle. The ends or edges of the anchor may be rounded or angulated. The anchor can be moved into a required position by pulling the thread and fixed in this position by securing the thread to the apex of the heart.

The anchor having the shape of an elongated cylinder may be formed by a roll of an elastic sheet. The roll has the diameter smaller than the inner diameter of the hollow needle. The elastic sheet may have a different floor plan, preferably in a rectangle or oval shape. There are two apertures for thread passage in the middle of the elastic sheet. The elastic sheet is a plastic foil. The elastic sheet forming the roll is adapted to resume its original flat shape.

The anchor may have a length in the range from 0.5 to 3 cm, a diameter in the range from 0.05 to 1 cm and the inner diameter of the aperture in the range from 0.005 cm to 1 cm, preferably the length is in the range from 0.5 to 1.5 cm, the diameter is in the range from 0.05 to 0.25 cm and the inner diameter of the aperture is in the range from 0.005 cm to 0.2 cm.

Invention also provides a method of use of the above described anchor during a mini-invasive surgery, that comprises:
a) inserting a hollow needle through a small incision between the ribs through the apex of the heart into the left ventricle to pierce the posterior leaflet or the anterior leaflet of the mitral valve,
b) pushing the anchor provided with the thread through the hollow needle by means of a guide wire,
c) pulling out the hollow needle,
d) positioning of the posterior leaflet or the anterior leaflet of mitral valve into the physiological position by moving the anchor by pulling the thread,
e) fixing of the posterior leaflet or the anterior leaflet in the physiological position by fixing the anchor to the apex of the heart using the thread.

All steps are performed under the echocardiographic control.

In case of using the anchor made from an elastic sheet the method comprises additional steps of: rolling up the elastic sheet into the roll before pushing it through the hollow needle, and unrolling the elastic sheet to resume its flat shape by pulling the thread after passing through the hollow needle.

### Brief description of the drawings

The invention is further explained using the drawings, where:

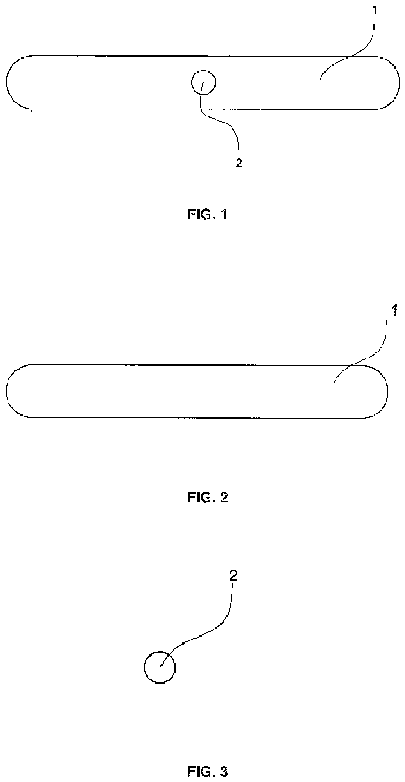

Fig. 1 shows a front view of the anchor according example 1,
Fig. 2 shows a top view of the anchor according example 1,
Fig. 3 shows a side view of the anchor according example 1,

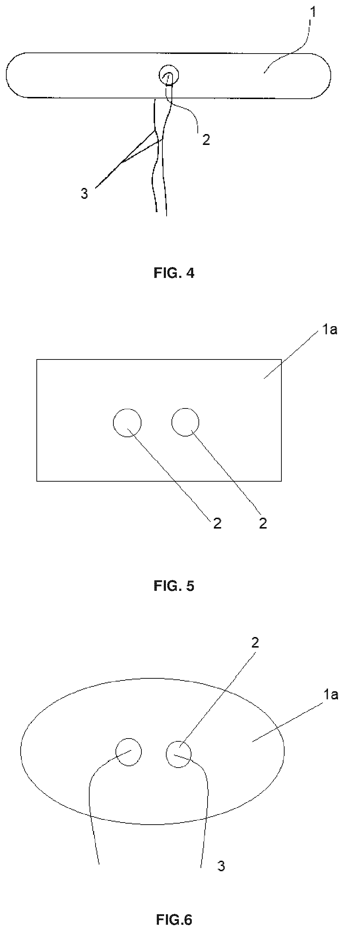

Fig. 4. shows the anchor according example 1 with a thread,
Fig. 5 shows a top view of a rectangular elastic sheet according example 2,
Fig. 6 shows a top view of an oval elastic sheet according example 2 with a thread,

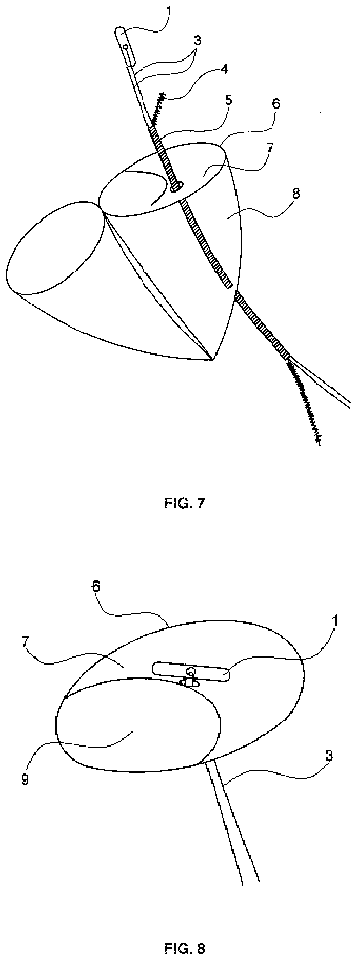

Fig. 7 shows a schematic introduction of the anchor into the mitral valve,
Fig. 8 shows schematically the positioning of the anchor on the mitral valve and attachment thereof.

### Examples

### Example 1

The anchor 1 has a shape of an elongated cylinder, which can pass through a hollow needle 5 by means of a guide wire 4. The diameter of the elongated cylinder is smaller than the inner diameter of the hollow needle 5, and the ends of the cylinder are rounded. The anchor 1 is provided with one aperture 2 in the middle of its length through which a surgical thread 3 passes. The surgical thread 3 is used to transfer the anchor and fix it in the required position. This exemplary anchor 1 has length of 1 cm, a diameter of 0.2 cm and the inner diameter of the aperture 2 of 0.1 cm. The anchor 1 is made from metal.

### Example 2

The anchor 1 has a shape of an elongated cylinder and it is formed by a roll of an elastic sheet 1a. The elastic sheet 1a is a plastic foil having a plan view in a rectangular shape, provided with two apertures 2 in the middle through which a surgical thread 3 passes. The surgical thread 3 is used to position the anchor 1 and fix it in the required position. The roll of elastic sheet 1a has its diameter smaller then the inner diameter of the hollow needle 5. Its length ranges from 0.5 to 3 cm, its width ranges from 0.05 to 1 cm and the inner diameter of the aperture 2 ranges from 0.005 cm to 1 cm.

### Example 3

Method of using the anchor 1 according to invention has following steps:
The hollow needle 5 is inserted into the left ventricle 8 through a small incision between the ribs and through the apex of the heart to pierce the posterior leaflet 7 and/or the anterior leaflet 9 of the mitral valve.

The anchor 1 having the thread 3 passing through the aperture 2 is then pushed through the hollow needle 5 by means of a guide wire 4. The hollow needle 5 is then pulled back and removed.

The anchor 1 is pulled towards the apex of the heart using the thread 3, which positions the posterior mitral valve leaflet 7 and/or the anterior mitral valve leaflet 9 in the physiological position.

The mitral valve leaflet 7 and/or the anterior leaflet 9 is fixed in the physiological position by securing the anchor 1 to the apex of the heart using the thread 3. The anchor 1 remains on the atrial side of the mitral valve leaflets. All steps are performed under the echocardiographic control.

In case of using the anchor 1 according to Example 2 the method comprises additional steps of: rolling up the elastic sheet 1a into the roll before pushing it through the hollow needle 5, and unrolling the elastic sheet 1a by pulling the thread 3 to resume its flat shape after passing through the hollow needle 5.

### REFERRALS:

- 1: anchor
- 1a: elastic sheet
- 2: aperture
- 3: thread
- 4: guide wire
- 5: hollow needle
- 6: mitral valve annulus
- 7: posterior leaflet of mitral valve
- 8: left ventricle
- 9: anterior leaflet of mitral valve

## Claims

1. Anchor (1) to repair a mitral valve prolapse by fixing mitral valve leaflet in the physiological position **characterized in that** it has an elongated shape, which is adapted for longitudinal passage through a hollow needle (5) by means of a guide wire (4) and it is provided with at least one aperture (2) for passing of a thread (3).

2. Anchor (1) according to claim 1, **characterized in that** the elongated shape is a cylinder, an ellipsoid or a prism, with at least one aperture (2) in the middle or its length.

3. Anchor (1) according to claim 2, **characterized in that** its ends or edges are rounded or angulated.

4. Anchor (1) according to claim 2, **characterized in that** it has the shape of the elongated cylinder made by a roll of an elastic sheet (1a).

5. Anchor (1) according to claim 4, **characterized in that** the elastic sheet (1a) has a floor plan in a rectangle or oval shape.

6. Anchor (1) according to claim 5, **characterized in that** there are two apertures (2) in the middle of the elastic sheet (1 a).

7. Anchor (1) according to claim 5, **characterized in that** the elastic sheet (1a) is adapted to resume its flat shape.

8. Anchor (1) according to any of the previous claims, **characterized in that** it is adapted to be transferred by pulling the thread (3) and fixed in a position by securing the thread (3).

9. Anchor (1) according to any of the previous claims, **characterized in that** its length is in the range from 0.5 to 3 cm, its width is in the range from 0.05 to 1 cm and the inner diameter of the aperture (2) is in the range from 0.005 cm to 1 cm, preferably its length is in the range from 0.5 to 1.5 cm, its width is in the range from 0.05 to 0.25 cm and the inner diameter of the aperture (2) is in the range from 0.005 cm to 0.2 cm.

10. Anchor (1) according to any of the previous claims according to claim 1, **characterized in that** the width of the anchor (1) is smaller than the inner diameter of the hollow needle (5).

11. Method of using the anchor (1) according to any of claims 1 to 9 to repair a mitral valve prolapse, **characterized in that** it comprises:
a) inserting the hollow needle (5) through a small incision between the ribs through the apex of the heart into the left ventricle (8) to pierce the posterior leaflet (7) and/or the anterior leaflet (9) of the mitral valve,
b) pushing the anchor (1) provided with the thread (3) through the hollow needle (5) by means of a guide wire (4),
c) pulling out and removal of the hollow needle (5),
d) positioning of the posterior leaflet (7) or the anterior leaflet (9) by moving the anchor (1) towards the apex of the heart by pulling the thread (3),
e) fixing of the leaflet(s) (7) and/or (9) in the physiological position by securing the anchor (1) to the apex of the heart using the thread (3),
wherein all steps are performed under the echocardiographic control.

12. Method according to claim 9, using the anchor (1) according to any of claims 4 -7, **characterized in that** it further comprises:
a step of rolling up the elastic sheet (1a) into the roll before passing through the hollow needle (5), and
a step of unrolling the elastic sheet (1 a) to resume its a flat shape after passing through the hollow needle (5).
